(19) **Europäisches Patentamt / European Patent Office / Office européen des brevets**

(11) **EP 1 550 374 B1**

(12) **EUROPEAN PATENT SPECIFICATION**

(45) Date of publication and mention
of the grant of the patent:
**18.06.2014 Bulletin 2014/25**

(51) Int Cl.:
***A23C 19/08*** (2006.01)     ***A23C 19/082*** (2006.01)

(21) Application number: **03751340.5**

(22) Date of filing: **03.10.2003**

(86) International application number:
**PCT/JP2003/012747**

(87) International publication number:
**WO 2004/032642 (22.04.2004 Gazette 2004/17)**

(54) **PROCESSED CHEESE PRODUCTS AND PROCESS FOR PRODUCING PROCESSED CHEESE**

SCHMELZKÄSEPRODUKTE UND VERFAHREN ZUR HERSTELLUNG VON SCHMELZKÄSE

PRODUITS DE FROMAGE FONDU ET PROCEDE DE PRODUCTION DE FROMAGE FONDU

(84) Designated Contracting States:
**AT BE BG CH CY CZ DE DK EE ES FI FR GB GR HU IE IT LI LU MC NL PT RO SE SI SK TR**

(30) Priority: **09.10.2002 JP 2002295719**

(43) Date of publication of application:
**06.07.2005 Bulletin 2005/27**

(73) Proprietor: **MEIJI CO., LTD.**
**Koutou-ku**
**Tokyo**
**136-8908 (JP)**

(72) Inventors:
• **ODA, Munehiro,**
**c/o Meiji Dairies Corporation**
**Odawara, Kanagawa 250-0862 (JP)**
• **AIZAWA, Sigeru,**
**c/o Meiji Dairies Corporation**
**Odawara, Kanagawa 250-0862 (JP)**
• **UCHIDA, Masayuki,**
**c/o Meiji Dairies Corporation**
**Odawara, Kanagawa 250-0862 (JP)**
• **SUZUKI, Masayuki,**
**c/o Meiji Dairies Corporation**
**Odawara, Kanagawa 250-0862 (JP)**
• **SASE, Manabu,**
**c/o Meiji Dairies Corporation**
**Oadawara, Kanagawa 250-0862 (JP)**

(74) Representative: **Isarpatent**
**Patent- und Rechtsanwälte**
**Postfach 44 01 51**
**80750 München (DE)**

(56) References cited:
**WO-A1-97/18718**          **JP-A- 2003 033 136**
**US-A1- 2002 182 301**

• **ITO TADASHI ET AL.: 'Cheese chu no angiotensin henkan koso inhibitor' MEDICINE AND BIOLOGY vol. 115, no. 6, 1987, pages 375 - 377, XP002977491**
• **OKAMOTO A. ET AL.: 'Angiotensin I converting enzyme inhibitory activities of various fermented foods' BIOSCI. BIOTECH. BIOCHEM. vol. 59, no. 6, 1995, pages 1147 - 1149, XP002955071**
• **MEISEL H ET AL: "ACE-inhibitory activities in milk products", MILCHWISSENSCHAFT, VV GMBH VOLKSWIRTSCHAFTLICHER VERLAG. MUNCHEN, DE, vol. 52, no. 6, 1 January 1997 (1997-01-01), pages 307-311, XP002986735, ISSN: 0026-3788**
• **SAITO T ET AL: "Isolation and Structural Analysis of Antihypertensive Peptides That Exist Naturally in Gouda Cheese", JOURNAL OF DAIRY SCIENCE, AMERICAN DAIRY SCIENCE ASSOCIATION, US, vol. 83, no. 7, 1 July 2000 (2000-07-01), pages 1434-1440, XP026993388, ISSN: 0022-0302 [retrieved on 2000-07-01]**
• **GOMEZ-RUIZ JOSE ANGEL ET AL: "Angiotensin-converting enzyme-inhibitory peptides in Manchego cheeses manufactured with different starter cultures", INTERNATIONAL DAIRY JOURNAL, ELSEVIER APPLIED SCIENCE, BARKING, GB, vol. 12, no. 8, 1 January 2002 (2002-01-01), pages 697-706, XP002592802, ISSN: 0958-6946**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of technology

This invention relates to processed cheeses, more specifically processed cheeses of which inhibition activity of angiotensin converting enzyme (ACE) is enhanced, and methods for manufacturing processed cheeses.

Background of Invention

High blood pressure is a risk factor for cerebral strokes and heart disease and is remedied by diet therapy, lifestyle improvement, and moreover, drug therapy by administering hypotensive drugs. However, since drug therapy always produces side effects, it is better not to rely on drug therapy. On the other hand, it has recently been found that peptides existing in food contain hypotensive peptides that have the effect of preventing high blood pressure and maintaining blood pressure within the normal range (for example, see reference publications 1 through 3). The mechanism is that the said hypotensive peptides inhibit the functions of the angiotensin converting enzyme (ACE). ACE is an enzyme that converts angiotensin I, a peptide in blood, to angiotensin II with a pressor function, inactivates kinin with a hypotensive function, and promotes a pressor effect. Furthermore, beverages that contain the same hypotensive peptides existing in food and have excellent an antihypertensive effect are commercially available (see reference publication 4).

Hypotensive peptides are also present in cheese. Animal tests using rats have proved that cheese fed to them prevents high blood pressure. It can be said that, if eating cheese daily is effective, then high blood pressure can be prevented more naturally than by ingesting beverages containing hypotensive peptides. Thus, the inventors have measured the ACE inhibitory activity of processed cheeses, which are commercially available, to obtain the results shown in table 1.

Table 1

ACE inhibitory activity of processed cheeses available in the market

| | ACE inhibitory activity (units per gram) |
|---|---|
| 6P cheese manufactured by Company A | 212 |
| 6P cheese (1) manufactured by Company B | 223 |
| 6P cheese (2) manufactured by Company B | 208 |
| Sliced cheese (1) manufactured by Company A | 199 |
| Sliced cheese (2) manufactured by Company A | 189 |
| Sliced cheese (1) manufactured by Company B | 156 |
| Sliced cheese (2) manufactured by Company B | 230 |
| Sliced cheese manufactured by Company C | 190 |

Reference publications other than patent document (1) T. Saito et al., Isolation and Structural Analysis of Antihypertensive Peptides That Exist Naturally in Gouda Cheese, Journal of Dairy Science, USA, Vol. 83, No. 7, 2000, p. 1434-1440 (2) H. Meisel et al., ACE-inhibitory activities in milk products, Milchwissenschaft, Germany, 52 (6) 1997, p. 307-311 (3) T. Ito et al., Angiotensin converting enzyme inhibitor in cheese, Medicine and Biology, Vol. 115, No. 6, 1987, p. 375-377 (4) Blood Pressure Seminar by Dr. Ameel, online, Calpis Co., retrieved on August 14, 2002, Internet, URL:http://ameel.calpis.co.jp/as/index.html

However, in Japan where cheese consumption is not as high as in Western countries, it can be predicted that it may be difficult to make daily intake of a certain amount of cheese a custom. In addition, excessive consumption of cheese may adversely promote high blood pressure due to the salt in cheese. For example, daily intake of soft drinks for high blood pressure prevention is limited to 5000 units converted to the ACE inhibitory activity. In order to supply this amount by consuming the commercially available cheese shown in table 1, the daily target intake is 22-32 g. This amount is far larger than the Japanese average intake of approximately 5.5 g. From these situations, it can be said that it would be very difficult for the current consumption of processed cheese to contribute to the prevention of high blood pressure.

This invention is made considering these situations. The purpose of the invention is to provide processed cheeses with an antihypertensive effect greater than conventional cheeses so that a small intake brings a sufficiently large antihypertensive effect.

Disclosure of the Invention

Processed cheeses according to this invention have a characteristic that angiotensin converting enzyme inhibitory activity is 350 units per gram or more. In addition, the processed cheeses according to the invention have a characteristic that the said cheeses use natural cheese having angiotensin converting enzyme inhibitory activity of 420 units per gram or more as raw materials. Moreover, the processed cheeses according to the invention have a characteristic that the sodium content of the said cheeses is 990 mg or less per 100 g of processed cheese. Furthermore, the processed cheeses according to the invention have a characteristic that the potassium content of the said cheeses is between 80 mg and 150 mg per 100 g of processed cheese.

A manufacturing method for the processed cheeses according to the invention, that is a manufacturing method for processed cheeses having angiotensin converting enzyme inhibitory activity of 350 units per gram or more, has a characteristic that the processed cheeses use at least one type of natural cheese with angiotensin converting enzyme activity of 420 units per gram or more as raw materials.

In addition, a manufacturing method for the processed cheeses according to the invention has a characteristic that the processed cheeses use low salt or unsalted natural cheese as natural cheese for a raw material in order to make the sodium content of the obtained processed cheese 990 mg or less per 100 g of processed cheese in the said manufacturing method.

Moreover, a manufacturing method for the processed cheeses according to the invention has a characteristic that the processed cheeses use potassium salt as molten salt in order to make the sodium content of the obtained processed cheese 990 mg or less per 100 g of processed cheese and, in these cases, to make the potassium content of the obtained processed cheese between 80 mg and 150 mg per 100 g of processed cheese in each of the said manufacturing methods.

Best Mode for Carrying out the Invention

The purpose of this invention is to provide processed cheeses that demonstrate an excellent antihypertensive effect with ACE inhibitory activity of 350 units per gram or more and to achieve an antihypertensive effect with an amount of cheese that can be taken regularly. Total daily intake of ACE inhibitory activity is about 5000 units if the same amount as that of the said beverage is considered to be required. As said, the average cheese consumption of the Japanese public is approximately 5.5 g. If it is assumed that the total daily intake of ACE inhibitory activity is achieved by consuming cheese, a cheese product 5.5 g in size is too small, and small as an amount from the viewpoint of the balance of lactoprotein or calcium intake. On the other hand, although cheese of which the weight per piece is 20-25 g is commercially available, this amount is too large in comparison with the average daily consumption of the Japanese. Therefore, even taking an increase in cheese consumption in the future into consideration, daily cheese intake is considered to be around 10-15 g. Thus the ACE inhibitory activity (specific activity) of processed cheeses is required to be 333-500 units per gram. Therefore, in order to achieve the purpose of the invention, ACE inhibitory activity of at least 350 units per gram, preferably 500 units per gram or more, and more preferably 700 units per gram or more is required. It is a matter of course that the cheese consumption of Western people is greater than that of the Japanese, and this specific activity has a value that can produce a sufficient antihypertensive effect. The ACE inhibitory activity in this invention is defined by values measured in the method described in Embodiments.

The processed cheeses according to the invention are products using natural cheese in processed cheese, cheese food, cheese spread, cheese dip, and cheese dessert as a main raw material.

In order to manufacture processed cheeses having a strong antihypertensive effect, it is necessary to select raw material natural cheese from natural cheeses with a strong antihypertensive effect. In this case, when considering general composition examples and deactivation in the manufacturing processes, the calculated value of ACE inhibitory activity of product processed cheeses is 357 units per gram, namely approximately 350 units per gram if a raw material cheese with ACE inhibitory activity of 420 units per gram is used. Thus processed cheese of 350 units per gram or more is obtained using a raw material cheese with ACE inhibitory activity of 420 units per gram or more. Therefore, the ACE inhibitory activity of the raw material natural cheese used in the invention is 420 units per gram or more, preferably 500 units per gram or more, and more preferably 700 units per gram or more.

In addition, in manufacturing processed cheeses, a combination of multiple types of cheese is often used as the raw material cheese. The purpose is to adjust flavor, adjust the degree of ripening, alleviate quality variation of each raw material cheese, and so on. Also in this invention, multiple types of raw material cheese can be combined to make the ACE inhibitory activity of total raw material cheese 420 units per gram or more, preferably 500 units per gram or more, and more preferably 700 units per gram or more. The types of cheese to be combined have no special limitation. For example, cheddar cheeses, such as Cheddar, Cheshire, Colby and Monterey jack; gouda type cheeses, such as Gouda, Samson and Maribo; Edam cheeses; extremely hard cheeses, such as Parmesan, Romano and Granapadano; Swiss cheeses, such as Emmental and Gruyeres; white mold cheeses, such as Camembert and Brie; blue mold cheeses, such as Stilton, Roquefort, Gorgonsola and Dana blue; cheeses of which the surface is ripened by bacteria, such as limburger; unripened cheeses, such as cream cheese, Mascarbone, Cottage and Mozarella; and whey cheeses such as ricotta can be used.

An amount of hypotensive peptide in cheese is different in each cheese. It varies depending on the types of cheese and the curing time. According to the data for commercially available raw material cheese measured by the inventors, an amount of peptide contained in unripened cheeses (fresh cheeses such as cottage cheese, cream cheese and quark, as well as unripened curd) is small because of the low degree of proteolysis, leading to a small antihypertensive effect. Not only that, it was found that the antihypertensive effect greatly varies depending on manufacturing methods (manufacturers) even if the type of cheese and the curing time are the same in ripened cheeses. The curing time, which is only a guideline, does not provide sufficient information for fully evaluating the strength of the antihypertensive effect. Thus it is necessary to select a raw material cheese after measuring the antihypertensive effect of each cheese. A part of the data of ACE inhibitory activity of natural cheese measured by the inventors is shown in table 2. Table 2 shows that cheddar cheese produced in New Zealand and Emmental cheese produced in Switzerland have a peculiarly large antihypertensive effect as examples. Therefore, in order to achieve the purpose of the invention, it is preferable to use cheddar cheese produced in New Zealand, Emmental cheese produced in Switzerland, or a combination of both, because these cheeses with a peculiarly large antihypertensive effect have ACE inhibitory activity of at least 420 units per gram. In addition, even when using these cheeses, it is preferable to use those having ACE inhibitory activity of 500 units per gram or more, and more preferably 700 units per gram or more.

TABLE 2
ACE inhibitory activity of raw material natural cheese

| | Number of maturity months | ACE inhibitory activity (units per gram) |
|---|---|---|
| Domestic Gouda cheese | 1 | 151 |
| Domestic Gouda cheese | 6 | 244 |
| Gouda cheese produced in Netherlands | 5 | 229 |
| Gouda cheese produced in Netherlands | 8 | 227 |
| Gouda cheese produced in New Zealand | 3 | 149 |
| Gouda cheese produced in New Zealand | 6 | 173 |
| Cheddar cheese produced in New Zealand | 7 | 709 |
| Cheddar cheese produced in New Zealand | 17 | 1141 |
| Cheddar cheese produced in Canada | 6 | 236 |
| Cheddar cheese produced in Canada | 13 | 277 |
| Parmesan cheese produced in Australia | 12 | 100 |
| Parmesan cheese produced in Australia | 20 | 93 |
| Emmental cheese produced in Switzerland | 9 | 525 |

On the other hand, it is well known that sodium acts to raise blood pressure. For example, processed cheese contains sodium from salt (content of 1.1-1.5 wt% in the product) and molten salt(content of 2-2.5 wt% in the product) such as phosphate, and citrate. Therefore, reduction of sodium in processed cheese products can further increase an antihypertensive effect of peptides. Sodium content of standard processed cheese is 1100 mg per 100 g of cheese (according to the Japan Food Content Table Ver. 5). In addition, cheese containing sodium at 980 mg or less per 100 g is classified as low-salt sodium food, so such a cheese is preferable for preventing high blood pressure. Therefore, the sodium content of processed cheese product is adjusted to 990 mg or less per 100 g of cheese.

Although a smaller amount of sodium content is better, a certain amount of sodium is necessary from the viewpoint of flavor and protection from bacteria. Moreover, since molten salt is also an essential additive for emulsification of processed cheeses, it is difficult to stop using molten salt. Therefore, it is preferable to properly combine unsalted natural cheese and low-salt natural cheese as a part of the raw material processed cheese to reduce the sodium content. Further, the molten salt is sometimes called an emulsifier.

Sodium from molten salt can be reduced by substituting potassium salt for a part of the sodium. Since potassium functions to lower blood pressure, substitution by potassium salt is an effective method for achieving the purpose of the invention. However, since potassium salt has a peculiar bitter taste, the potassium content is adjusted to 150 mg or less per 100 g of cheese. In this connection, since the potassium content of normal processed cheese is around 60 mg per 100 g of cheese, it is necessary to increase the amount of potassium in order to reduce the sodium content. Thus, in this invention, the potassium content is between 80 mg per 100 g of cheese and 150 mg per 100 g of cheese.

The invention is specifically explained by describing the embodiments. However, the invention is not restricted by the following embodiments:

(EMBODIMENT 1)

Processed cheese was prepared using cheddar cheese (curing time of 12 months and ACE inhibitory activity of 940 units per gram) produced in New Zealand under the following processes:

[Composition]

| | |
|---|---|
| Cheddar cheese produced in New Zealand | 8.5 kg |
| Molten salt (sodium polyphosphate) | 0.1 kg |
| Molten salt (disodium hydrogen phosphate) | 0.1 kg |
| Water (including steam for heating) | 1.3 kg |

[Process]

Raw material cheese was coarsely shredded in advance using a meat chopper. All the raw materials were fed to a kettle type kneader with a volume of 20 liters (however the amount of water does not include that of steam for heating), and was agitated at a speed of 120 rpm while blowing in steam for about 10 minutes to heat the materials to 85°C. Two hundred grams each of fluid molten cheese were packed into containers, which were tightly closed to cool in a refrigerator at 5°C for one night.

This processed cheese was found to have good flavor and texture. In addition, the measured value of ACE inhibitory activity of 820 units per gram demonstrated that processed cheese having ACE inhibitory activity sufficiently higher than commercially available processed cheese can be manufactured.

[Measurement of ACE inhibitory activity]

Pretreatment of samples is performed as follows: Filtered pure water (500 ml) is added to 100 g of shredded pieces of cheese and agitated by a mixer for five minutes. Then the material is subjected to centrifugal separation at 7000 rpm for 30 minutes to split the water layer and remove siltation and oil from the upper layer. The water layer is filtered to obtain the sample liquid.

Measurement of ACE inhibitory activity is performed as follows: Sample liquid is neutralized using 1 N of sodium hydroxide. The neutralized 0.04 ml of sample liquid is mixed with 0.1 ml of enzyme liquid (Angiotensin Converting Enzyme:2 units per milliliter) to heat the mixture to 37°C. Then the 0.1 ml matrix layer (Hippuryl-His-Leu; N-Benzoyl-Gly-His-Leu) is added and sufficiently agitated. The liquid is kept at 37°C for 60 minutes to allow a reaction. After the reaction, 0.13 ml of 1 N hydrochloric acid is added and sufficiently agitated to stop the reaction. Then 0.85 ml of ethyl acetate is added, shaken for one minute, and subjected to centrifugal separation at 3000 rpm for 10 minutes. Supernatant (0.7 ml) is collected to remove the solvent by a centrifugal evaporator (for about 30 minutes). Then, 0.5 ml of distilled water is added to this to dissolve residual substances to measure absorbance at a wave length of 228 nm. ACE inhibitory activity (units per gram) is calculated using the following equation 1. In the equation, $A$ is absorbance of the control when an enzyme is used, $B$ is that of the control when no enzyme is used, $C$ is that of the sample liquid when an enzyme is used, and $D$ is that of the sample liquid when no enzyme is used. Water was used as a control instead of the sample liquid. Equation 1 Inhibitory activity

$$\text{Inhibitory activity (unit/g)} = \frac{(A-B)-(C-D)}{(A-B)} \times \frac{100}{50} \times \frac{1}{0.04} \times 6$$

(EMBODIMENT 2)

Cheese spread was prepared using cheddar cheese produced in New Zealand (curing time of seven months and ACE inhibitory activity of 709 units per gram) and Emmental cheese produced in Switzerland (curing time of nine months and ACE inhibitory activity of 525 units per gram) as raw material cheese. Composition is as follows. The process is the same as Embodiment 1.

[Composition]

| | |
|---|---|
| Cheddar cheese produced in New Zealand | 3.0 kg |
| Emmental cheese produced in Switzerland | 3.0 kg |
| Vegetable oil (rapeseed oil) | 1.0 kg |
| Molten salt (sodium polyphosphate) | 0.15 kg |
| Molten salt (sodium citrate) | 0.05 kg |
| Water (including steam for heating) | 2.8 kg |

This cheese was found to have good flavor and texture. The measured value of ACE inhibitory activity of 370 units per gram demonstrated that cheese spread of which ACE inhibitory activity is sufficiently increased in comparison with processed cheese and cheese spread, both commercially available, can be manufactured.

(EMBODIMENT 3)

Processed cheese was prepared using cheddar cheese produced in New Zealand (curing time of 17 months, ACE inhibitory activity of 1141 units per gram and salt content of 2.0 wt%) and low-salt cheddar cheese produced in New Zealand (curing time of 0.5 months, ACE inhibitory activity of 140 units per gram and salt content of 0.5 wt%) as raw material cheese. Composition is as follows. The process is the same as Embodiment 1.

[Composition]

| | |
|---|---|
| Cheddar cheese produced in New Zealand | 6.5 kg |
| Low-salt cheddar cheese produced in New Zealand | 1.5 kg |
| Molten salt (sodium polyphosphate) | 0.2 kg |
| Water (including steam for heating) | 1.8 kg |

This cheese was found to have good flavor and texture. The measured value of ACE inhibitory activity of 760 units per gram demonstrated that processed cheese of which ACE inhibitory activity is sufficiently increased in comparison with commercially available processed cheese can be manufactured. Sodium content was 950 mg% (950 mg of sodium in 100 g of cheese).

(EMBODIMENT 4)

Processed cheese was prepared using cheddar cheese produced in New Zealand (curing time of seven months, ACE inhibitory activity of 709 units per gram, and salt content of 2.0 wt%) and Emmental cheese produced in Switzerland (curing time of nine months, ACE inhibitory activity of 525 units per gram and salt content of 1.8 wt%) as raw material cheese. Composition is as follows. The process is the same as Embodiment 1.

[Composition]

| | |
|---|---|
| Cheddar cheese produced in New Zealand | 6.0 kg |
| Emmental cheese produced in Switzerland | 2.0 kg |
| Molten salt (sodium polyphosphate) | 0.15 kg |
| Molten salt (potassium polyphosphate) | 0.05 kg |
| Water (including steam for heating) | 1.8 kg |

This cheese was found to have good flavor and texture. The measured value of ACE inhibitory activity of 760 units per gram demonstrated that processed cheese of which ACE inhibitory activity is sufficiently increased in comparison with commercially available processed cheese can be manufactured. Sodium content was 850 mg% and potassium content was 120 mg%.

Industrial feasibility

This invention provides processed cheese of which an antihypertensive effect is sufficiently increased in comparison with conventional processed cheese and an intake of the cheese according to this invention naturally controls blood pressure. In particular, processed cheese, of which the sodium content is reduced, controls the intake of sodium and further contributes to controlling blood pressure. In other words, the processed cheese with an antihypertensive effect according to this invention is utilized as a specific health food for assisting in the maintenance of normal blood pressure and as functional food capable of standing for an antihypertensive effect.

**Claims**

1. A manufacturing method for processed cheeses having angiotensin converting enzyme inhibitory activity of 350 units per gram or more, wherein at least one type of natural cheese having angiotensin converting enzyme inhibitory activity of 420 units per gram or more is used as a raw material, and wherein low salt or unsalted natural cheese is used as a raw material, and the sodium content of the obtained processed cheese is 990 mg or less per 100 g of processed cheese, and wherein potassium salt is used as raw material molten salt, wherein potassium content of the obtained processed cheese is between 80 mg and 150 mg per 100 g of processed cheese.

2. A processed cheese manufactured by the method of claim 1.

**Patentansprüche**

1. Herstellungsverfahren für Schmelzkäse, der eine Hemmaktivität für Angiotensinkonvertierendes Enzym von 350 Einheiten pro Gramm oder mehr aufweist, wobei zumindest ein Typ eines natürlichen Käses mit einer Hemmaktivität für Angiotensin konvertierendes Enzym von 420 Einheiten pro Gramm oder mehr als Ausgangsmaterial verwendet wird, und wobei salzarmer oder ungesalzener natürlicher Käse als Ausgangsmaterial verwendet wird, und der Natriumgehalt des gewonnenen Schmelzkäses 990 mg oder weniger pro 100 g Schmelzkäse beträgt, und wobei Kaliumsalz als Ausgangsmaterial-Schmelzsalz verwendet wird, wobei der Kaliumgehalt des gewonnenen Schmelzkäses zwischen 80 mg und 150 mg pro 100 g Schmelzkäse beträgt.

2. Schmelzkäse, der durch das Verfahren nach Anspruch 1 hergestellt worden ist.

**Revendications**

1. Procédé de fabrication de fromages fondus ayant une activité inhibitrice de l'enzyme convertissant l'angiotensine de 350 unités par gramme ou plus, dans lequel au moins un type de fromage naturel ayant une activité inhibitrice de l'enzyme convertissant l'angiotensine de 420 unités par gramme ou plus est utilisé comme matière brute, et dans lequel du fromage naturel faiblement salé ou non salé est utilisé comme matière brute, et la teneur en sodium du fromage fondu obtenu est de 990 mg ou moins par 100 g de fromage fondu, et dans lequel du sel de potassium est utilisé comme sel fondu de matière brute, dans lequel la teneur en potassium du fromage fondu obtenu est entre 80 et 150 mg par 100 g de fromage fondu.

2. Fromage fondu fabriqué par le procédé selon la revendication 1.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. SAITO et al.** Isolation and Structural Analysis of Antihypertensive Peptides That Exist Naturally in Gouda Cheese. *Journal of Dairy Science, USA,* 2000, vol. 83 (7), 1434-1440
- **H. MEISEL et al.** ACE-inhibitory activities in milk products. *Milchwissenschaft, Germany,* 1997, vol. 52 (6), 307-311
- **T. ITO et al.** Angiotensin converting enzyme inhibitor in cheese. *Medicine and Biology,* 1987, vol. 115 (6), 375-377
- **DR. AMEEL.** Blood Pressure Seminar. Calpis Co, 14 August 2002